# EUROPEAN PATENT APPLICATION

(11) **EP 3 364 366 A1**
(43) Date of publication of application: **22.08.2018**
(21) Application number: 16859797.9
(22) Date of filing: 25.10.2016
(51) Int. Cl.: G06Q 50/22

(54) **EXAMINATION ORDER PROCESSING DEVICE AND EXAMINATION SYSTEM USING SAME**

(30) Priority: 26.10.2015 JP 2015209905
(71) Applicant: Horiba, Ltd., Kyoto-shi, Kyoto 601-8510 (JP)
(72) Inventor: DEJIMA, Yutaka, Kyoto-shi Kyoto 601-8510 (JP)
(74) Representative: Isarpatent
(86) International application number: PCT/JP2016/081599
(87) International publication number: WO 2017/073567

(57) **Abstract**

Provided are a test order processing apparatus capable of communicating with a test apparatus and a test system using same. The test order processing apparatus 1 contains a test order receiving part 110 that receives a test order containing plural test items, a linking information-retaining part 120 that forms linking information for linking an individual test item to a test apparatus appropriate therefor, according to the linking information, a test request sending part 130 that sends each test item to a test apparatus appropriate therefor, a test result receiving part 140 that receives the test results sent back from each test apparatus and forms a test report, and a test report sending part 150 that sends the test report to a sender of the test order.

## Description

### [Technical Field]

The present invention relates to a test order processing apparatus for causing a test apparatus appropriate for a test relating to a test item contained in a test order formed by a doctor or the like to perform the test and a test system using the test order processing apparatus.

### [Background Art]

In recent years, various test apparatuses (also called automatic analysis apparatus and the like) for automatically performing measurements of specimen (blood, urine, feces and the like), collected from a test subject, with respect to given test items have been developed (e.g., patent document 1 and the like). In such test apparatus, a specimen is automatically taken into the apparatus from the specimen container by merely setting a specimen container such as a blood collection tube, a urine collection tube or the like and measurement and analysis with respect to given test item(s) are automatically performed, and measurement results are output. In many test apparatuses, moreover, measurement and analysis with respect to not only a single test item but also measurements and analyses with respect to many test items can be performed.

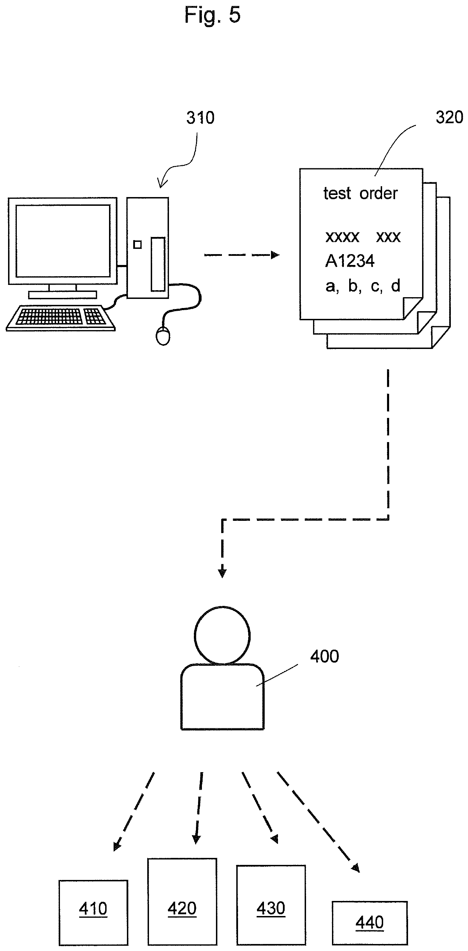

On the other hand, in recent years, many medical institutions introduce management systems using computers, and an increasing number of medical institutions introduce electronic medical records instead of paper medical charts (medical examination records) hand-written by doctors, as schematically shown in Fig. 5. It is also possible to form, during operation to form electronic medical record, a test order necessary for diagnosing patients and test subjects.

A test order contains at least identifying information of the test order itself and test items necessary for diagnosis. In the embodiment of Fig. 5, as a simple example, identifying information A1234 of the test order itself and full name of the test subject: xxxx xxx and test items a, b, c, d requesting specimen testing are contained in the test order 320.

In the test department, many test apparatuses for performing various tests are disposed. Fig. 5 shows four test apparatuses 410, 420, 430 and 440, as examples. These test apparatuses can perform tests (analysis and measurement) of the test items in a test order (hereinafter to be also referred to as test apparatus appropriate for test item).

For example, when a test order 320 contains a test item requiring a specimen testing, a test staff (clinical laboratory technician etc.) 400 in a test department collects a specimen from the test subject (or selects a specimen container to which the same identifying information as the above-mentioned identifying information is affixed from specimen containers containing specimens collected separately in advance) according to the content of the test order, selects a test apparatus appropriate for the test items a, b, c, d in the test order 320, and performs specimen testing using the test apparatus. Then, the test staff sends the test results output from the test apparatus as a test report back to the doctor.

### [Document List]

### [Patent document]

patent document 1: JP-A-2014-215210

### [SUMMARY OF THE INVENTION]

### [Problems to be Solved by the Invention]

However, the present inventors studied in detail the issue of test order and the test performance status (particularly the state of specimen testing) in the test department, and noted the following problems to be improved.

The problem is one in the above-mentioned work when an appropriate test apparatus is selected based on the items described in the test order and the test is performed. The test order is printed out in some department. In a typical example, as illustrated in Fig. 5, a test order 320 formed by a doctor using a terminal computer 310 is printed out as a paper document from a printer in a medical examination room, and delivered to a test staff 400 in the test department. The test staff 400 watches the test order 320 described in the delivered paper document and selects, from many test apparatuses, a test apparatus appropriate for the test items described in the test order, and inputs at least the identifying information relating to the test order into the test apparatus. When one test apparatus deals with many test items, only the necessary test items may be selected and input.

As mentioned above, an operation in which a test staff selects a test apparatus appropriate for test items in a test order and inputs identifying information and the like for each test apparatus problematically involves a possibility of error such as input error and the like. It also problematically requires labor to input. These problems become particularly noticeable in a specimen testing in which many test items need to be distributed to many kinds of test apparatuses.

More particularly, in the practice of the above-mentioned conventional test order, a test staff inputs identifying information and the like into the test apparatus, during which the input items may be erroneously input. When the identifying information is erroneously input and a specimen (specimen of other person) matching the erroneous identifying information is present, the specimen of other person is analyzed to cause a serious problem. When a specimen matching the erroneous identifying information is not present, analysis is not performed, thus resulting in a waste of time. When the test items are erroneously input, collected specimens are consumed for unnecessary analysis, and a requested test cannot be performed (or specimen needs to be recollected), thus causing problems and the like. There is also a possibility of omission of test (requested test items cannot be tested due to the failure to input a part of test items to be inputted) or redundant test.

In addition, an operation to select a test apparatus and an operation to input identifying information into a test apparatus per se place a burden on the test staff. Some of the test apparatuses for specimen testing can process many kinds of test items by themselves. Such test apparatus also requires an operation to select only the necessary test items from the test items the apparatus can process. As a result, selection error may occur and the operation places a burden on the test staff.

Furthermore, an operation to integrate test results sent back from each test apparatus, to which the test items were distributed, into one test report may involve errors and also requires labor.

The problem of the present invention is to reduce an information input work into a test apparatus when test items contained in a test order are processed by a test apparatus.

### [Means of Solving the Problems]

The present inventors have conducted intensive studies in an attempt to solve the aforementioned problem and found that provision of a test order processing apparatus capable of automatically sending test items contained in a test order to a test apparatus appropriate for the test items basically obviates an operation in which a test staff selects a test apparatus appropriate for test items in a test order and inputs identifying information for each test apparatus and solves the above-mentioned problem, which resulted in the completion of the present invention.

The main constitution of the present invention is as follows.
[1] A test order processing apparatus for causing a test apparatus to perform a test relating to a test item contained in a test order, the test order processing apparatus comprising:
   a test order receiving part that receives the test order containing one or more test items from a sender of the test order;
   a linking information-retaining part that retains information linking said one or more test items contained in the test order to a test apparatus appropriate therefor; and
   a test request sending part that refers to the linking information and sending, according to the linking information, a test request for each test item to a test apparatus appropriate for the test item.
[2] The test order processing apparatus according to [1], further comprising:
   a test result receiving part that receives test results sent back from the test apparatus in response to the test request;
   a test report forming part that forms a test report based on the test results in response to the test order; and
   a test report sending part that sends the test report to a given recipient.
[3] The test order processing apparatus according to [2], wherein
   the test order contains plural test items, the number of the test apparatus is in plurality, and
   the test report forming part forms one test report using the test results sent back from the plural test apparatuses in response to the test order.
[4] The test order processing apparatus according to any of [1] to [3], wherein the sender of the test order is a computer capable of communicating with the test order processing apparatus.
[5] The test order processing apparatus according to any of [2] to [4], wherein the linking information contains information that links one test item to plural test apparatuses, whereby said one test item is sent as a test request from the test request sending part to plural test apparatuses,
   the test result receiving part is configured to transmit information of a test apparatus that first sent back the test results or test start information, from among the plural test apparatuses to which said one test item was sent, to the test request sending part, and
   the test request sending part is configured to send an instruction to delete the test request sent to the test apparatuses other than the test apparatus that first sent back the test results or test start information.
[6] A testing system comprising the test order processing apparatus according to any of [1] to [5] and one or more test apparatuses connected to the test order processing apparatus, wherein said one or more test apparatuses
   comprise a display screen;
   are configured to display test request information showing each test request, sequentially received from the test order processing apparatus, on the display screen in the order of receipt thereof; and
   are configured to erase the test request information relating to the test request from the display screen once analysis relating to the test request is completed and sequentially display other remaining test request information.

### [Effect of the Invention]

Intervention of the test order processing apparatus of the present invention between the sender of a test order and a test apparatus enables automatic distribution and sending of all test items contained in the test order to appropriate test apparatuses. Therefore, a test staff no longer needs to select a test apparatus or input identifying information and the like relating to a test order into a test apparatus, which in turn basically eliminates errors in the above-mentioned input or selection and solves the above-mentioned conventional problems.

The display screen of each test apparatus displays information of the test request relating to test items distributed by the test order processing apparatus (test request information). A test staff only needs to set a specimen corresponding to the identifying information and start the test function of the test apparatus according to the test request information displayed on the display screen of the test apparatus.

This usefulness becomes particularly noticeable in specimen testing. This is because specimen testing often includes plural test items in a test order and such plural test items need to be distributed to plural test apparatuses.

In a preferable embodiment of the present invention, the test order processing apparatus further has a test result receiving part, a test report forming part, and a test report sending part. In this case, not only a function to automatically distribute and send test items of a test order to each test apparatus is afforded, but also the test results sent back from each test apparatus can be automatically sent as a test report to given recipients (e.g., sender of test order, computer of other department and the like). Therefore, a test staff is no longer required to transcribe the test results from a test apparatus as a test report in response to the test order, and an error associated therewith is also eliminated.

Particularly, when plural test apparatuses are necessary for processing plural test items, the test order processing apparatus functions to send test requests to plural test apparatuses, automatically integrate test results sent back from the plural test apparatuses into one test report, and automatically send same to a given recipient. Thus, the errors made by and burden of operation placed on the test staff are further improved. Such usefulness also becomes particularly noticeable in specimen testing for the above-mentioned reasons.

In a test system using the test order processing apparatus, as described in detail below, each test apparatus connected to the test order processing apparatus is configured to display each test request sequentially received from the test order processing apparatus on the display screen in the order of receipt as the test request information. It is also configured to erase the test request information relating to the test request from the above-mentioned display screen once analysis relating to each test request is completed and sequentially display test request information of other remaining test requests. Using the system configured as above, a test staff only needs to always take note of the test request information displayed first on the display screen of the test apparatus, set a specimen appropriate therefor on the test apparatus and start the test. Therefore, the errors made by and burden of operation placed on the test staff are further reduced.

### [Brief Description of the Drawings]

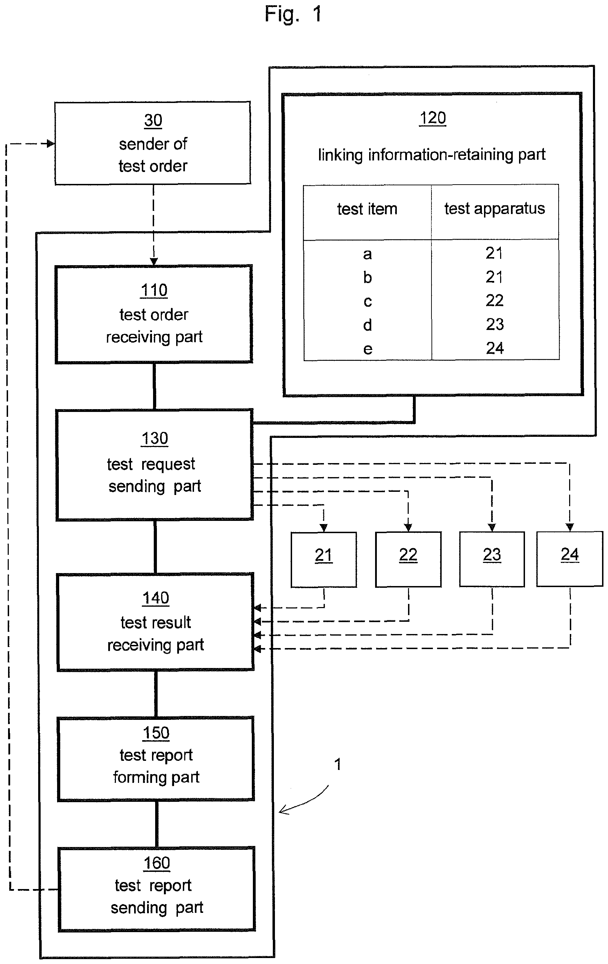

Fig. 1 is a block diagram showing a preferable embodiment of the constitution of the test order processing apparatus of the present invention, which is also a block diagram showing one embodiment of the constitution of a test system using the test order processing apparatus. The communication between the test order processing apparatus of the present invention and outside apparatuses (sender of test order, test report recipient, test apparatus) is shown with a broken line. In the embodiment of Fig. 1, a test result receiving part 140, a test report forming part 150, a test report sending part 160 are further provided in addition to the basic constitution of the present invention (test order receiving part 110, linking information-retaining part 120, test request sending part 130). In the embodiment of Fig. 1, the sender of the test order is also the recipient of the test report.
Fig. 2 is a block diagram showing a preferable embodiment of the constitution of the test order processing apparatus of the present invention, which is also a block diagram showing one embodiment of a preferable constitution of a test system using the test order processing apparatus.
Fig. 3 is a flowchart illustrating the flow of processing of the test order performed by the test order processing apparatus of the present invention.
Fig. 4 explains a preferable operation of a test apparatus connected to the test order processing apparatus of the present invention, or a test apparatus constituting the test system of the present invention. It illustrates changes on the display screen of the test apparatus.
Fig. 5 is a block diagram showing conventional connection state of doctor's terminal computer and a terminal computer of a test department and the state of use of the test apparatus disposed in the test department.

### [Description of Embodiments]

The present invention is explained in detail in the following by referring to Examples.

Fig. 1 is a block diagram showing an embodiment of the constitution of the test order processing apparatus of the present invention, which is also a block diagram showing one embodiment of the constitution of a test system using the test order processing apparatus. As shown in Fig. 1, the test order processing apparatus 1 is an apparatus for causing a test apparatus to perform a test relating to a test item contained in a test order sent from a sender 30 of the test order, and contains at least a test order receiving part 110, a linking information-retaining part 120, and a test request sending part 130. The test order receiving part 110 receives the test order containing one or more test items from a sender 30 of the test order. The linking information-retaining part 120 retains information linking said one or more test items contained in the test order to a test apparatus appropriate therefor. The test request sending part 130 refers to the above-mentioned linking information retained by the linking information-retaining part 120 and sends, according to the linking information, a test request for each test item to a test apparatus appropriate for the test item.

The linking information retained by the linking information-retaining part 120 illustrated in Fig. 1 links in advance test items a, b to test apparatus 21, test item c to test apparatus 22, test item d to test apparatus 23, and test item e to test apparatus 24. When, for example, a test order contains test items a, b, c, d, the test request sending part 130 refers to the linking information and sends a test request to, of the test apparatuses 21 - 24 in the Figure, test apparatuses 21 - 23 appropriate for the test items a, b, c, d. The test request is mentioned later.

With the above constitution, all test items a, b, c, d contained in the test order are automatically distributed and sent to appropriate test apparatuses. As a result, input error by a test staff is basically eliminated and a burden of operation is drastically reduced.

In a preferable embodiment of the test order processing apparatus, as shown in Fig. 1, a test result receiving part 140, a test report forming part 150 and a test report sending part 160 are further provided in addition to the above-mentioned test order receiving part 110, linking information-retaining part 120 and test request sending part 130.

The test result receiving part 140 receives the test results sent back from the test apparatus in response to the test request. In the present specification, the test result receiving part 140 is described as an independent part to facilitate understanding of the function of each part. The test request sending part 130 and the test result receiving part 140 can also be understood as one sending and receiving part that integrally performs bidirectional communication with the test apparatus (sending of test request, reception of test results, sending of other additional command, reception of response and the like).

The test report forming part 150 forms a test report based on the test results to the primary test order. The test report sending part 160 sends back the formed test report to a given recipient (e.g., sender 30 of test order and the like).

With the above constitution, plural test items contained in a test order are distributed to plural test apparatuses. When respective test results are sent back from the test apparatuses, the test results are automatically integrated into one test report and automatically sent back to a given recipient. Thus, error in work reports by a test staff and a burden of operation are drastically improved.

The sender of the test order is not particularly limited and may be any as long as it is a computer capable of communicating with the test order processing apparatus. It may be a sender according to the network of the system such as a terminal computer used by a doctor or the like (from which test order is issued), a computer of a management department that receives a test order from the terminal computer and sends the test order to the test order processing apparatus or the like.

On the other hand, the given recipient to which the test report is sent is not particularly limited and may be any as long as it is a computer capable of communicating with the test order processing apparatus. It may be a recipient predetermined according to the network of the system or flow of a test report such as a terminal computer used by a doctor or the like, a computer of a management department that receives a test report from the test order processing apparatus and sends the test report to a terminal computer of a doctor etc., or the like. In the network system of a general medical institution, the sender (issuing source) of a test order is often a recipient of the test report (e.g., when a test order is sent from a doctor's terminal computer (issuing source) and the test report is sent back to the doctor's terminal computer and the like). However, the sender of the test order and the recipient of the test report for the test order processing apparatus may be different from each other.

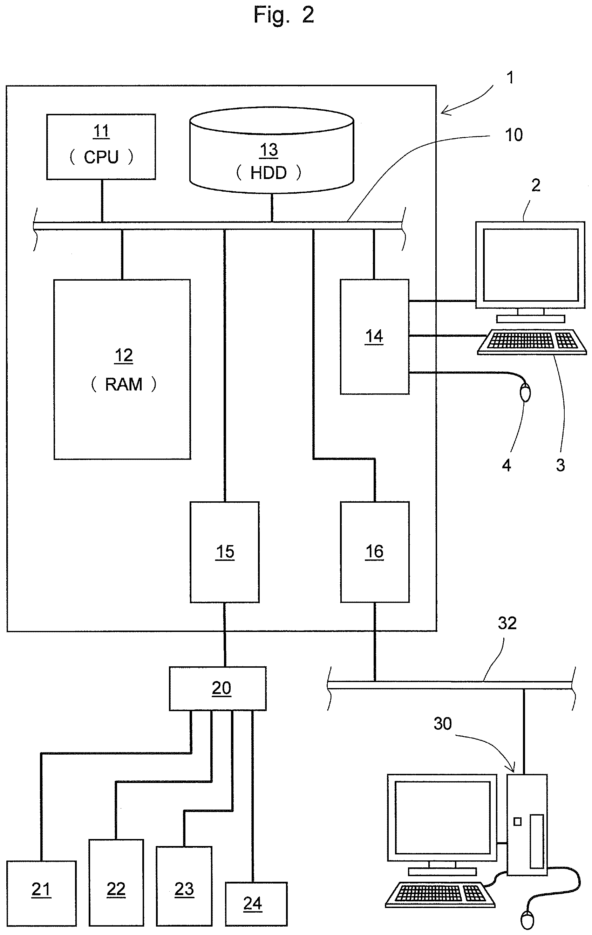

Each part constituting the test order processing apparatus (test order receiving part, linking information-retaining part, test request sending part, and test result receiving part, test report making part, test report sending part and the like added in preferable embodiment mentioned above) may each be constructed using a combination of an electron circuit, an electric circuit and an independent processing apparatus. In a preferable embodiment, each of these parts may be, as shown in Fig. 2, constituted using a computer and a program to be executed by the computer.

In the following, a preferable embodiment and an effect of each part are explained by illustrating an embodiment in which the test order processing apparatus is constituted using a computer. The constitution of each part shown below can be performed partially or entirely by a combination of an electron circuit, an electric circuit, an independent processing apparatus and the like in place of a computer.

Fig. 2 is a block diagram showing a preferable embodiment of the test order processing apparatus and test system, and shows an embodiment in which the test order processing apparatus is constituted using a computer. In the embodiment shown in Fig. 2, the test order processing apparatus 1 intervenes between the terminal computer 30 used by a doctor or the like and plural test apparatuses 21 - 24, and can also be said a host computer for automatically distributing a test order (further preferably, for returning the test results as one automatically summarized test report).

The basic architecture itself as a computer of the test order processing apparatus 1 shown in Fig. 2 may be the same as that of a conventionally-known computer. As shown in Fig. 2, the computer as the test order processing apparatus 1 has a constitution in which a central processing unit (CPU) 11 and a random access memory (RAM) 12 as a main memory are connected by a data bus 10. Preferably, a hard disk drive (HDD) 13 is connected to impart a CPU 11 with a storage region having large capacity. The hard disk drive may be appropriately substituted by other storage device such as a solid-state drive and the like. The hard disk drive 13 functions as a linking information-retaining part 120 in Fig. 1. In the embodiment of Fig. 2, display device 2, keyboard 3 and mouse 4 are connected to the data bus 10 via an interface 14 for an input/output device. For example, these are used when an administrator changes or updates as necessary the content etc. of the program executed by the test order processing apparatus. In addition, such input/output device may be used for inputting/outputting (display of results) during operation of other various programs executed by the test order processing apparatus.

In the embodiment of Fig. 2, plural test apparatuses (test apparatuses 21 - 24 as examples as in Fig. 1) are communicatably connected via an interface 15 for an external device. The test order processing apparatus 1 can send test request data to respective test apparatuses 21 - 24. In a more preferable example, the test results data sent back from each test apparatus can be received. In the embodiment of Fig. 2, each test apparatus is connected to the test order processing apparatus 1 by USB (universal serial bus) interface 15 and hub 20 to enable bidirectional communication. The kind and total number of the test apparatus are not limited, and the kind and connection mode (wired, wireless) of the interface are not limited.

In the embodiment of Fig. 2, the sender of a test order and the recipient of the test report are each a terminal computer 30 used by a doctor. The test order processing apparatus can be connected to the terminal computer 30 used by a doctor via a communication line 32 of a network interface 16 and LAN (may be internet and the like), and has a constitution in which a test order sent by the terminal computer 30 can be received and an appropriate test request can be sent to each test apparatus.

A test order may contain one or more test items, and the usefulness of the present invention becomes noticeable when many test items are contained.

The sender of a test order may be, for example, as shown in Fig. 2 as a typical example, other computer communicatably connected to the test order processing apparatus such as the terminal computer 30 used by a doctor and the like. In this case, the test order receiving part 110 is constituted such that a test order data is received from other computer and identifying information and test items contained in the test order are received via a network interface and the like.

The sender of the test order may also be contained in the test order processing apparatus itself. For example, the test order processing apparatus may also be a terminal computer used by a doctor and the like, and electronic medical record program and the like, and a program that issues a test order may be executed by the test order processing apparatus. In this case, the program that issues a test order is constituted to transfer the data of the test order to a test order receiving part 110 of the host program, and the test order receiving part 110 is constituted to receive the data of the test order.

The test subject may be not only a human but also an animal other than human. When the test subject is a human, the test subject may include not only patients having a disease but also healthy subjects not having a disease. For example, in a medical examination and the like, any medical examinee is the subject irrespective of the presence or absence of a disease and the test data of given test items are obtained.

In the embodiment of Fig. 2, the test order processing apparatus has at least a test order receiving part 110, a linking information-retaining part 120 and a test request sending part 130, and further has a test result receiving part 140, a test report forming part 150 and a test report sending part 160 in a preferable embodiment. Each of these parts is a part wherein the test order processing apparatus functions by the program executed by the test order processing apparatus (hereinafter to be also referred to as the host program). In other words, the host program is constituted to make the computer as a test order processing apparatus function as at least a test order receiving part 110 or a test request sending part 130, and further as a test result receiving part 140, a test report forming part 150 or a test report sending part 160.

Each of the above-mentioned function (operation content) achieved by executing the host program may contain not only a computing operation performed by CPU and the main memory but also function achieved by each hardware constituting the test order processing apparatus and outside hardware under the direction by the host program. In addition, a part of each function mentioned above may be executed by an outside apparatus, which is connected to the test order processing apparatus, under the control of the host program.

The function of each part of the test order processing apparatus is explained in more detail below by following a flowchart illustrating processing in each part.

In the test order in one embodiment for explanation, which is similar to the conventional embodiment shown in Fig. 5, the information identifying the test order itself is A1234 and test items are 4 items of a, b, c, d. To facilitate identification by a test staff, the name of the test subject (xxxx xxx) is contained as the identifying information of the test subject (identifying information of the test subject may be identification number accorded to the test subject).

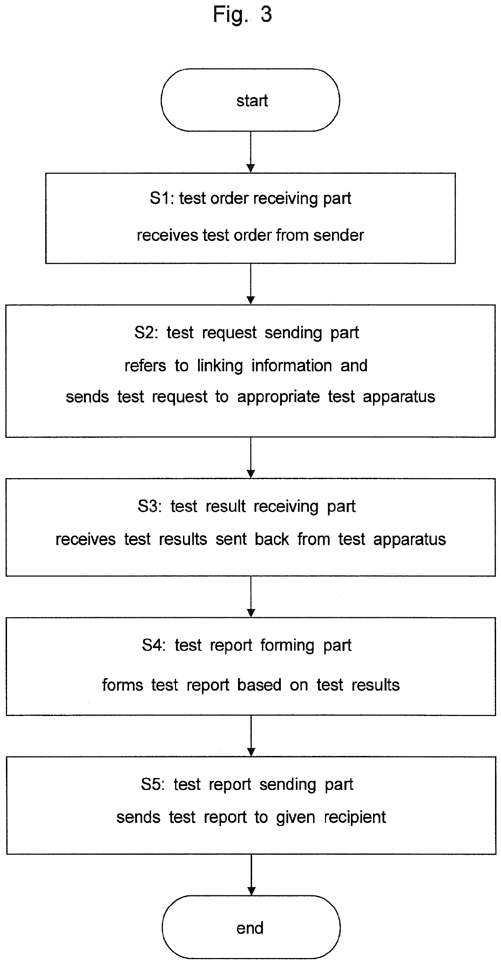

As shown in the flowchart of Fig. 3, in step s1, a test order receiving part 110 receives a test order from a sender 30. Each data of the test order in the constitution example of Fig. 2 is sent from other computer (e.g., terminal computer operated by doctor) 30 through a communication line 32 and received by the test order processing apparatus through a network interface 16. The test order receiving part 110 accepts each data of the test order as the test order data that should be processed.

In step s2, a test request sending part 130 refers to the linking information stored in a linking information-retaining part 120 (hard disc drive 13 in Fig. 2) and send test items a, b, c, d contained in the test order as a test request to a test apparatus appropriate therefor according to the linking information. In the embodiment of Fig. 2, a hard disc drive 13 as a linking information-retaining part stores linking information as database that links various test items a, b, c, d, e to be contained in the test order to respective test apparatuses 21 - 24 appropriate for the test items. Similar to the explanation in Fig. 1, test items a, b are associated with test apparatus 21, test item c is associated with test apparatus 22, test item d is associated with test apparatus 23, and test item e is associated with test apparatus 24, in advance.

Therefore, the test request sending part 130 that has referred to the linking information issues a test request to each test apparatus to cause testing of test items a, b contained in the test order by a test apparatus 21, test item c by a test apparatus 22, and test item d by a test apparatus 23.

The test items are not particularly limited and may be those contained in various test orders such as test items relating to specimen testing using specimens (blood, urine, feces, cell and the like) collected from the test subject as the test targets, test items using the body of the test subject as the test target such as electrocardiographic measurement, ultrasonic diagnosis, radiographic inspection, CT scan and the like, and the like. As mentioned above, the usefulness of the present invention becomes particularly noticeable when the test items relate to specimen testing.

Examples of the test items relating to specimen testing include test items about hematological test, biochemical test, immunological test, genetic test, and the like. The specific test items recited below are not necessarily classified into only one of the above-mentioned tests and may be classified into, for example, both hematological test and biochemical test and the like.

Examples of test items relating to blood count include white blood cell count, red blood cell count, hemoglobin concentration, hematocrit value, average red blood cell volume, mean corpuscular hemoglobin, mean corpuscular hemoglobin concentration, platelet count, red blood cell distribution width, mean platelet volume, platelet distribution width, platelet crit, lymphocyte count, lymphocyte ratio, monocyte count, monocyte ratio, granulocyte count, granulocyte ratio, neutrophil count, neutrophil ratio, eosinophil count, eosinophil ratio, basophil count, basophil ratio, atypical lymphocyte count, atypical lymphocyte ratio, large juvenile cell count, large juvenile cell ratio, reactive protein concentration, blood glucose level, ammonia, sodium, potassium, chlorine and the like.

Examples of test items relating to hemoglobin A1c (HbA1c) include glycohemoglobin A1c, hemoglobin F (HbF), urine albumin, urine creatinine, urine AC ratio, high sensitivity C-reactive protein (HSCRP) concentration, cystatin and the like.

Examples of test items targeting urine include color, turbidity, glucose, protein, bilirubin, urobilinogen, pH, specific gravity, occult blood, ketone body, nitrite salt, leukocyte test, ascorbic acid, creatinine, µ albumin, p/c ratio (protein-creatinine ratio), a/c ratio (albumin-creatinine ratio), urea nitrogen, uric acid, total cholesterol, ammonia, neutral fat, total protein, albumin, total bilirubin, calcium, inorganic phosphorus, direct bilirubin, HDL cholesterol, magnesium, γ-glutamyl transpeptidase, AST (GOT), ALT (GPT), creatine kinase, lactic acid dehydrogenase, alkaline phosphatase, leucine aminopeptidase, CK-MB, choline esterase, amylase, lipase, sodium, potassium, chloride and the like.

Examples of biochemical test items include total protein, albumin, neutral fat, total cholesterol, LDL cholesterol, HDL cholesterol, AST (GOT), ALT (GPT), γ-glutamyl transpeptidase, alkaline phosphatase, lactic acid dehydrogenase, choline esterase, total bilirubin, direct bilirubin, leucine aminopeptidase, ammonia, amylase, lipase, urea nitrogen, creatinine, uric acid, glucose, creatine kinase, calcium, inorganic phosphorus, magnesium, hemoglobin A1c, sodium, potassium, chloride, ketone body, SAA, C-reactive protein (CRP), hemoglobin concentration, CK-MB, cCRP, fructosamine, rheumatoid factor quantification and the like.

Examples of genetic test items include gene test, chromosome test, DNA test, RNA test and the like.

As the test apparatus, an apparatus communicatably connected to the test order processing apparatus and capable of measurement and analysis relating to the above-mentioned test items can be used. Examples of test apparatus relating to specimen testing include blood cell counting apparatus, blood analyzing apparatus, concentration measuring apparatus, immunological measuring apparatus, genetical analyzing apparatus and the like. Only one test apparatus may be able to process only one test item, or one test apparatus may be able to process plural test items.

The connection between the test order processing apparatus and the test apparatus is not particularly limited, and any connection capable of data communication between a computer and an external apparatus such as connection via wired or wireless LAN, USB, RS-232C, GPIB, blue tooth (registered trade mark), internet, a combination of these and the like can be used.

The number of the test apparatuses that can be connected to the test order processing apparatus may be one or more. The usefulness of the present invention becomes more noticeable when the number is plural, particularly when a greater number of test apparatuses are connected.

As mentioned above, the linking information-retaining part 120 retains linking information in which the above-mentioned various test items are linked in advance to test apparatuses appropriate for any of the test items and communicatably connected to the test order processing apparatus. The linking information is preferably stored as the database (hereinafter to be also referred to as the linking database) to be referred to.

The linking database stores in advance all test items contained in a test order and all test apparatuses appropriate for any of the test items and communicatably connected to the test order processing apparatus, as the linking information in which they are linked in advance and in a form a computer can refer to. While the structure of the linking database is not particularly limited, a form permitting quick and easy reference by the test request sending part 130 such as table and the like is preferable.

To link each test item and a test apparatus appropriate therefor, an identifying code specific to each test item is preferably accorded. On the other hand, while a specific identifying code may be accorded to each test apparatus, existing identifying information specific to each test apparatus is preferably utilized. Examples of the existing identifying information specific to each test apparatus include vendor IDs, product IDs and the like when the test apparatus is USB equipment, I/O port address, apparatus-specific identifying number and the like when the test apparatus is an apparatus connected via a general-purpose interface such as RS-232C and the like, and IP address, an apparatus-specific identifying number and the like when the test apparatus is an apparatus connected via various networks.

In addition, the test order processing apparatus preferably further contains a linking database modifying part (not shown) to be a tool for modifying the link between test items and test apparatuses appropriate for the test items stored in the linking database. In the embodiment of Fig. 2, the linking database modifying part is a computer program for linking database modification, which is constituted to perform deletion of abolished test items and test apparatuses, addition of new test items and new test apparatuses, linking newly-added test item and existing test apparatus, linking newly-connected test apparatus and existing test items and the like through input/output devices (2, 3, 4) in the linking database.

The test request sending part 130 sends a test request relating to test items contained in the test order to the test apparatus appropriate for the test items according to the linking information stored in the linking information-retaining part 120 (hard disc drive 13 in the embodiment of Fig. 2).

The test request relating to test items is one kind of test order sent to each test apparatus from the test order processing apparatus. The test request is accorded with specific identifying information. This aims to link the test request to be sent and the below-mentioned test results to be sent back. As the identifying information specific to the test request, the identifying information of the primary test order (e.g., identifying information A1234 in the embodiment of Fig. 5) is preferably used. As a result, each test apparatus can be linked to the identifying information accorded to the specimen container and one test report corresponding to the primary test order can be formed from the test results sent back from each test apparatus. The test request may contain both the specific identifying information used for communication between the test order processing apparatus and the test apparatus, and the identifying information of the test order to be displayed to a test staff. In addition, the test request may further contain the full name of the test subject (e.g., xxxx xxx in the embodiment of Fig. 5) to enhance distinction performance by the test staff.

When a test apparatus to which a test request is to be sent can only perform a test relating to only one test item, it is not necessary to send the test item as a test request to such test apparatus. For example, the command of the test request may be replaced by sending identifying information specific to the test request. In addition, when a test apparatus to which a test request is to be sent can perform tests relating to plural test items and the test apparatus is used to always perform all tests that the apparatus can perform, the command of the test request may be replaced by sending identifying information specific to the test request as in the above case.

On the other hand, when a test apparatus can perform tests relating to many test items and perform tests relating to some of the test items, it is preferable to send, as a test request, identifying information specific to the test request and the necessary test items.

From the above points, a test request relating to test items contains identifying information specific to the test request and contains, where necessary, information relating to the test items.

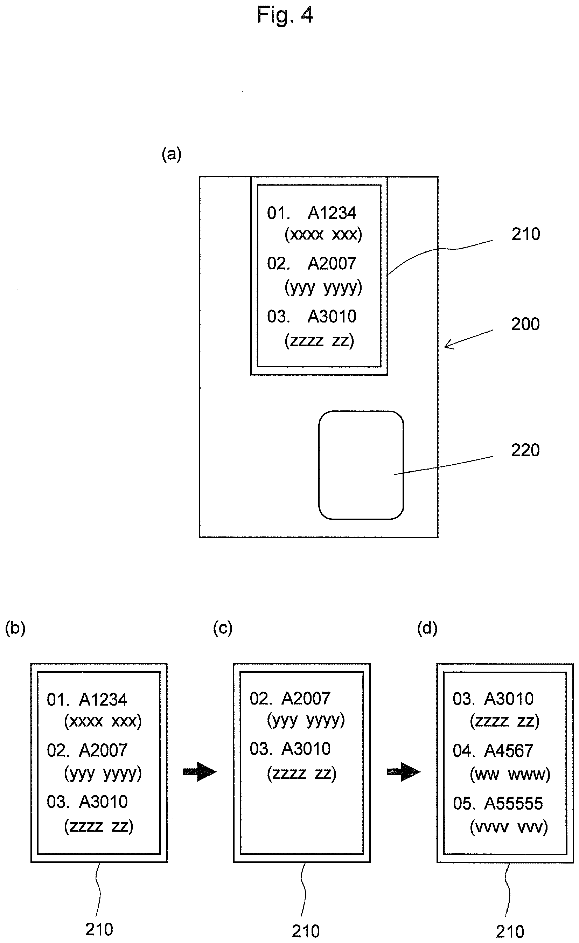

Each test apparatus to be connected to the test order processing apparatus is, as shown in Fig. 4(a), preferably constituted to display, on each display screen, information relating to the test request received (test request information). The information relating to the test request to be displayed on a display screen of each test apparatus preferably displays at least the identifying information of the primary test order so that a test staff can correctly link the test request to a specimen (or test subject).

In the embodiment of Fig. 4, the number showing the order of receipt of the test requests (01 and the like), identifying information of the test order (A1234 and the like), and the full name of the test subject (xxxx xxx and the like) are displayed on a display screen 210 of a test apparatus 200. In the embodiment of Fig. 4, an openable and closable set part 220 to set a specimen container is formed on the front face of the test apparatus 200.

It is preferable to impart the test apparatus with a function to read and collate the identifying information described on the specimen container set and the identifying information specific to the test request (preferably, identifying information of the primary test order). This enables detection of a set of incorrect specimen containers. Moreover, when an apparatus received a first test request and a second test request, it is possible to configure the test apparatus to process the second test request even when a specimen container corresponding to the second test request has been set.

When the analysis and measurement relating to the test request are completed, the test apparatus sends back the test results to the test order processing apparatus in response to the test request. The test results preferably contain, in addition to the resulting test values, identifying code showing the test items of the test results and the identifying information specific to the primary test request (preferably, identifying information of the primary test order). In this way, a test report in response to the primary test order can be restored from the test results of divided test items.

Steps S3 - S5 shown in the flowchart in Fig. 3 are preferable processing steps to be added to the test order processing apparatus. It is a processing step for receiving the test results sent back from the test apparatus to which the test request was sent, automatically forming a test report from the test results, and sending same back to the predetermined recipient.

In step S3, the test result receiving part 140 receives the test results sent back from the test apparatus as a response to the primary test request. Respective test results contain the identifying information specific to the test request (preferably, identifying information of the primary test order).

In step S4, the test report forming part 150 forms a test report by using the received test results and the identifying information specific to the primary test order.

In step S5, the test report sending part 160 sends the above-mentioned test report to a given recipient (sender 30 of test order and the like, predetermined recipient), whereby a series of processing of the test order are completed.

In Steps S3 - S5, a test report is automatically formed from the test results sent back from each test apparatus, and automatically sent back to a given recipient, whereby the errors made by and burden of operation placed on the test staff are drastically improved.

The test report formed by the test report forming part 150 preferably contains the identifying information of the primary test order. This enables the test report sending part 160 to send a test report in response to the test order from a given recipient, and finally, the sender and issuing source of the test order can receive a test report corresponding to the primary test order it sent (or issued).

In one embodiment of the present invention, one test item may be linked to plural test apparatuses. For example, when the same kind of plural test apparatuses are connected to the test order processing apparatus, or when the connected plural test apparatuses are different from each other in the kind but the test items they can process partly overlap, the test request sending part 130 may send the same test request x1 to the plural test apparatuses and distribute a single test item redundantly to plural test apparatuses.

In this embodiment, the information of the same test request x1 is displayed on each display screen of plural test apparatuses (A1, A2, A3: not shown). A test staff can select a suitable test apparatus (e.g., A1) such as a test apparatus with less test requests and the like, set the specimen container and start the analysis or measurement.

When the analysis or measurement is completed, as mentioned above, the test apparatus A1 sends back the test results to the test order processing apparatus as a response to the test request.

On the other hand, the test request sending part 130 sends information that the same test request x1 was sent to plural test apparatuses A1, A2, A3 to the test result receiving part 140. The test result receiving part 140 conveys, to the test request sending part 130, information that the test apparatus A1 out of those test apparatuses A1, A2, A3 first sent back the test results. The test request sending part 130 that received the information instructs test apparatuses (A2, A3) other than the test apparatus A1 to delete the initially-sent test request x1. Here, the test request sending part 130 and the test result receiving part 140 are explained as if they jointly control the test apparatus. It is also understood that one sending and receiving part integrally formed by the test request sending part 130 and the test result receiving part 140 sends and receives data, command signals (response signals) and the like with outside test apparatuses.

By this constitution, even when one test item is redundantly sent to plural test apparatuses A1, A2, A3, the redundant instructions are deleted once one test is completed.

In a variation of the above-mentioned embodiment, it may be configured that a test staff selects a test apparatus (e.g., A1), sets a specimen container and starts analysis and measurement, at which time point the test apparatus A1 sends information of the start of the test relating to test request x1 to a test result receiving part 140, and a test request sending part 130 instructs test apparatuses (A2, A3) other than the test apparatus A1 to delete the initially-sent test request x1.

As shown in Fig. 2, by communicatably connecting plural test apparatuses 21 - 24 to the test order processing apparatus 1 of the present invention, a preferable test system is constituted. The test order processing apparatus 1 may be communicatably connected to other computer 30 as a sender of a test order.

The contents of the operation of the test order processing apparatus and each test apparatus are as mentioned above. As shown in Fig. 4, each test apparatus has a display screen 210, and is configured to display the test request information showing respective test requests sequentially received from the test order processing apparatus 1 on the display screen 210 in the order of receipt. In the embodiment of Fig. 4(b), three pieces of test request information (1: A1234), (2: A2007), (3: A3010) are displayed including the full name of the test subject. The serial numbers 1, 2, 3 were given for easy explanation and are not essential.

In a preferable embodiment of the test apparatus, the test apparatus is configures to erase the test request information relating to the test request (e.g., first test request information (1: A1234) in Fig. 4(b)) from the display screen 210 once the analysis relating to each test request is completed and, as shown in Fig. 4(c), sequentially move up and display test request information (2: A2007), (3: A3010) relating to other remaining test requests. On the display screen shown in Fig. 4(d), as the analysis relating to the test request further proceeded, test request information (2: A2007) was deleted, the test request information (3: A3010) moved up, and test request information (4: A4567), (5: A5555) relating to a newly-received test request are displayed.

By such constitution, as mentioned above, a test staff only needs to always take note of the test request information displayed first on the display screen of the test apparatus, set a specimen appropriate therefor on the test apparatus and start the test. Therefore, the burden of operation placed on and errors in selection made by the test staff are further reduced.

### [Industrial Applicability]

According to the test order processing apparatus and test system using same of the present invention, all test items contained in the test order can be automatically distributed and sent to appropriate test apparatuses. Therefore, a test staff no longer needs to select a test apparatus or input identifying information and the like relating to a test order into a test apparatus, which in turn eliminates errors in the information input and reduces labor of input. In addition, when an operator such as a doctor and the like sends a test order by a tool such as electronic medical record and the like, it is no longer necessary to specify a test apparatus corresponding to the test item. Furthermore, an error in summarizing test results from each test apparatus into one test report does not occur and the labor thereof can also be reduced.

The test system of the present invention can be utilized not only as a system for diagnosis in medical institution but also a test system in any industry in which a test order is sent to a test department.

This application is based on a patent application No. 2015-209905 filed in Japan (filing date: October 26, 2015), the contents of which are incorporated in full herein.

### [Explanation of Symbols]

1 test order processing apparatus
2 display device
3 keyboard
4 mouse
10 data bus
11 central processing unit (CPU)
12 main memory (RAM)
13 hard disc drive
14 - 16 interface
110 test order receiving part
120 linking information-retaining part
130 test request sending part
140 test result receiving part
150 test report forming part
160 test report sending part
21 - 24 test apparatus
30 outside computer

## Claims

1. A test order processing apparatus for causing a test apparatus to perform a test relating to a test item contained in a test order, the test order processing apparatus comprising:
a test order receiving part that receives the test order containing one or more test items from a sender of the test order;
a linking information-retaining part that retains information linking said one or more test items contained in the test order to a test apparatus appropriate therefor; and
a test request sending part that refers to the linking information and sending, according to the linking information, a test request for each test item to a test apparatus appropriate for the test item.

2. The test order processing apparatus according to claim 1, further comprising:
a test result receiving part that receives test results sent back from the test apparatus in response to the test request;
a test report forming part that forms a test report based on the test results in response to the test order; and
a test report sending part that sends the test report to a given recipient.

3. The test order processing apparatus according to claim 2, wherein
the test order contains plural test items, the number of the test apparatus is in plurality, and
the test report forming part forms one test report using the test results sent back from the plural test apparatuses in response to the test order.

4. The test order processing apparatus according to any one of claims 1 to 3, wherein the sender of the test order is a computer capable of communicating with the test order processing apparatus.

5. The test order processing apparatus according to any one of claims 2 to 4, wherein the linking information contains information that links one test item to plural test apparatuses, whereby said one test item is sent as a test request from the test request sending part to plural test apparatuses,
the test result receiving part is configured to transmit information of a test apparatus that first sent back the test results or test start information, from among the plural test apparatuses to which said one test item was sent, to the test request sending part, and
the test request sending part is configured to send an instruction to delete the test request sent to the test apparatuses other than the test apparatus that first sent back the test results or test start information.

6. A testing system comprising the test order processing apparatus according to any one of claims 1 to 5 and one or more test apparatuses connected to the test order processing apparatus, wherein said one or more test apparatuses
comprise a display screen;
are configured to display test request information showing each test request, sequentially received from the test order processing apparatus, on the display screen in the order of receipt thereof; and
are configured to erase the test request information relating to the test request from the display screen once analysis relating to the test request is completed and sequentially display other remaining test request information.
